# EUROPÄISCHE PATENTSCHRIFT

(11) **EP 1 296 926 B1**
(45) Veröffentlichungstag und Bekanntmachung des Hinweises auf die Patenterteilung: **23.04.2008**
(21) Anmeldenummer: 01945338.0
(22) Anmeldetag: 04.07.2001
(51) Int. Cl.: C07C 67/08, C07C 29/16, C08K 5/12, C07C 31/125

(54) **VERFAHREN ZUR HERSTELLUNG VON GEMISCHEN VON DIESTERN DER PHTHALSÄURE MIT DECANOLEN UND TRIDECANOLEN**
METHOD FOR PRODUCING BLENDS OF PHTHALIC ACID DIESTERS, DECANOLS AND TRIDECANOLS
PROCEDE DE PREPARATION DE MELANGES DE DIESTERS DE L'ACIDE PHTALIQUE AVEC DES DECANOLS ET DES TRIDECANOLS

(30) Priorität: 05.07.2000 DE 10032580; 12.06.2001 DE 10128306
(43) Veröffentlichungstag der Anmeldung: 02.04.2003
(73) Patentinhaber: BASF SE, 67056 Ludwigshafen (DE)
(72) Erfinder: BREITSCHEIDEL, Boris, 67117 Limburgerhof (DE); SELBERDINGER, Richard, 67126 Hochdorf-Assenheim (DE); ROSSATO, Klaus, 67105 Schifferstadt (DE); STORZUM, Uwe, 67551 Worms (DE); HOLZMANN, Jürgen, 67071 Ludwigshafen (DE); DISTELDORF, Walter, 67157 Wachenheim (DE); ÜBLER, Christoph, 68161 Mannheim (DE); MORSBACH, Bernd, 67069 Ludwigshafen (DE); PETERS, Jarren, 68163 Mannheim (DE); GOLFIER, Günter, 67227 Frankenthal (DE)
(86) Internationale Anmeldenummer: PCT/EP2001/007637
(87) Internationale Veröffentlichungsnummer: WO 2002/002499

(56) Entgegenhaltungen:
- EP-A- 0 424 767
- WO-A-01/36356
- GB-A- 1 301 312
- US-A- 5 880 310

## Beschreibung

Die vorliegende Erfindung betrifft ein Verfahren zur Herstellung von Gemischen von Diestern der Phthalsäure mit Decanolen und Tridecanolen.

Weiterhin betrifft die vorliegende Erfindung derartige Gemische, deren Verwendung als Weichmacher für Formmassen sowie für deren Herstellung geeignete Gemische isomerer Tridecanole.

Langkettige Alkohole, z.B. C₁₀-, C₁₁- und C₁₃-Alkohole, werden in großem Umfang zur Herstellung von Weichmachern eingesetzt. Die Alkohole werden hierzu mit Polycarbonsäuren, wie insbesondere Phthalsäure, zu den entsprechenden Estern umgesetzt.

Wichtige Vertreter der Weichmacher aus der Klasse der Phthalsäureester sind die Diisodecylphthalate und die Diisotridecylphthalate. Sie werden vor allem bei der Herstellung von Kabelummantelungen, Leitungen und Rohren aus Polyvinylchlorid ("PVC") eingesetzt, etwa im Automobilbau.

Der Stand der Technik kennt bereits Phthalsäureester als Weichmacher, welche als Alkoholeinheiten C₁₀-Alkohole (Decanole) oder C₁₃-Alkohole (Tridecanole) aufweisen:

Die JP-A 07 179 699 und die JP-A 08 283 510 beschreiben Weichmacher für PVC-Filme, bei denen es sich um Diester der Phthalsäure mit einem Gemisch aus den C₁₀-Alkoholen 2-Propylheptanol und 4-Methyl-2-propylhexanol im Gewichtsverhältnis 88 : 12 bis 70 : 30 handelt. Die JP-A 08 301 295 beschreibt die Verwendung derartiger Ester als Weichmacher für PVC, in denen das Gewichtsverhältnis von 2-Propylheptanol und 4-Methyl-2-propylhexanol 100 : 0 bis 50 : 50 beträgt. Auch aus der JP-A 08 034 891 ist ein Weichmacher für PVC-Filme auf Basis von 2-Propylheptanol und 4-Methyl-2-propylhexanol bekannt und darüber hinaus ein solcher, der nur 2-Propylheptanol als Alkoholeinheit enthält.

Die JB 73/35705 und die JA 81/47443 lehren die Verwendung des Diesters von Phthalsäure mit Tridecanol als Weichmacher für Vinylchloridpolymere wie PVC.

Für den Fachmann auf dem Gebiet der PVC-Kunstoffe, der sich insbesondere mit der Herstellung von Folien, Kabeln und Leitungen aus diesem Material befasst, stellt sich immer häufiger die Aufgabe, Weichmacher einzusetzen, die einerseits die plastischen Eigenschaften dieser Kunststoffe verbessern, allen voran die Kälteelastiziät, aber andererseits auch nur wenig flüchtig sind. Die verminderte Flüchtigkeit trägt wesentlich dazu bei, dass die bei der Herstellung erzielte Elastizität des PVC-Kunststoffs über lange Zeit erhalten bleibt und dass der Weichmacher praktisch nicht an die Umgebung abgegeben wird.

Die bekannten Phthalsäureester-Weichmacher mit Decanolen oder Tridecanolen als Alkoholeinheit können in dieser Hinsicht jedoch noch nicht befriedigen.

Der vorliegenden Erfindung lag daher die Aufgabe zugrunde, Weichmacher auf Basis von Estern des Phthalsäureanhydrids mit Alkoholen von 10 und mehr Kohlenstoffatomen bereitzustellen, die dem geschilderten Nachteil abzuhelfen vermögen.

Demgemäß wurden ein Verfahren zur Herstellung von Gemischen von Diestern der Phthalsäure mit Decanolen und Tridecanolen gefunden, dass dadurch gekennzeichnet ist, dass man Phthalsäure oder ein reaktives Derivat der Phthalsäure mit einem Gemisch aus isomeren Decanolen und einem Gemisch aus isomeren Tridecanolen umsetzt.

Weiterhin wurden derartige Gemische sowie deren Verwendung als weichmacher für Formmassen gefunden.

Die erfindungsgemäßen weichmacher bewirken eine hohe Kälteelastizität und weisen eine niedrige Flüchtigkeit auf, die jene der Phthalsäureester von Decanolen allein oder Tridecanolen allein übertreffen.

Als Decanole sind aliphatische Monoalkohole mit 10 Kohlenstoffatomen bevorzugt, insbesondere die primären Alkanole, in denen die Alkylgruppen geradkettig oder verzweigt sein können, beispielsweise n-Decanol, die Methylnonanole wie 1-Methylnonanol, die Ethyloctanole wie 1-Ethyloctanol, die Propylheptanole wie 1-Propylheptanol oder die Methylpropylhexanole, jeweils einzeln oder im Gemisch, vor allem jeweils für sich allein 2-Propylheptanol und 4-Methyl-2-propylhexanol. Besonders bevorzugt sind Gemische der genannten Alkanole ("Isodecanole"), insbesondere diejenigen mit den CAS-Nummern 25339-17-7 und 93821-11-5. Ganz besonders bevorzugt sind Gemische aus zusammen überwiegend, vorzugsweise 80, vor allem 90 und insbesondere 95 Gew.-% 2-Propylheptanol und 4-Methyl-2-propylhexanol, und zwar im Verhältnis 99 : 1 bis 1 : 99, vorzugsweise 95 : 5 bis 50 : 50 und besonders bevorzugt 92 : 8 bis 88 : 12 Gew.-%.

Als Tridecanole sind aliphatische Monoalkohole mit 13 Kohlenstoffatomen bevorzugt, insbesondere die primären Alkanole, in denen die Alkylgruppen geradkettig oder verzweigt sein könnnen, beispielsweise n-Tridecanol, die Methyldecanole wie 1-Methyldodecanol oder die Ethylundecanole wie 1-Ethylundecanol, jeweils einzeln oder im Gemisch. Bevorzugt sind Gemische der genannten Alkanole ("Isotridecanole") und insbesondere diejenigen mit den CAS-Nummern 27458-92-0 und 68526-86-3. Besonders bevorzugt sind solche Isotridecanole, die nach dem folgenden Verfahren erhältlich sind:

In einem mehrstufigen Verfahren, das von einem Butene enthaltenden Kohlenwasserstoffgemisch ausgeht, werden in einem ersten Schritt die Butene zu einem Gemisch isomerer Octene und Dodecene dimerisiert. Als Hauptprodukt fallen dabei die Octene an, während die Dodecene mit einem Anteil von im Allgemeinen 5 bis 20 Gew.-%, bezogen auf den Reaktoraustrag, anfallen. Aus dem Reaktionsgemisch werden anschließend die Dodecene isoliert, zu den entsprechenden C₁₃-Aldehyden hydroformyliert und nachfolgend zu Isotridecanolen hydriert. Die Herstellung von Isotridecanolen durch die genannte Abfolge von Syntheseschritten ist an sich bekannt. Die erfindungsgemäß besonders bevorzugte Isotridecanole werden jedoch nur erhalten, wenn zumindest bei der Butendimerisierung, vorzugsweise bei der Butendimerisierung und der Hydroformylierung, spezielle, definierte Randbedingungen eingehalten werden.

So ist es bevorzugt, dass das Gemisch isomerer Dodecene durch Inkontaktbringen eines Butene enthaltenden Kohlenwasserstoffgemisches mit einem heterogenen Katalysator, der Nickeloxid enthält, erhalten wird. Vorzugsweise beträgt der iso-Butengehalt des Kohlenwasserstoffgemisches 5 Gew.-% oder weniger, insbesondere 3 Gew.-% oder weniger, besonders bevorzugt 2 Gew.-% oder weniger und am meisten bevorzugt 1,5 Gew.-% oder weniger, jeweils bezogen auf den Gesamtbutengehalt. Ein geeigneter Kohlenwasserstoffstrom ist der sogenannte C₄-Schnitt, ein Gemisch aus Butenen und Butanen, das in großen Mengen aus FCC-Anlagen oder Steamcrackern zur Verfügung steht. Als besonders bevorzugter Einsatzstoff wird das sogenannte Raffinat II verwendet, bei dem es sich um einen iso-Buten-abgereicherten C₄-Schnitt handelt.

Ein bevorzugtes Einsatzmaterial enthält 50 bis 100, vorzugsweise 80 bis 95 Gew.-% Butene und 0 bis 50, vorzugsweise 5 bis 20 Gew.-% Butane. Als allgemeiner quantitativer Rahmen läßt sich folgende Zusammensetzung der Butenfraktion anführen:
- 1-Buten: 1 bis 99 Gew.-%
- cis-2-Buten: 1 bis 50 Gew.-%
- trans-2-Buten: 1 bis 99 Gew.-%
- iso-Buten: bis zu 5 Gew.-%.

Als Katalysator kommen an sich bekannte Katalysatoren in Betracht, die Nickeloxid enthalten, wie sie z.B. von O'Connor et al. in Catalysis Today 6, (1990) S. 329 beschrieben sind. Es können geträgerte Nickeloxid-Katalysatoren verwendet werden, wobei als Trägermaterialien Kieselsäure, Tonerde, Aluminosilicate, Aluminosilicate mit Schichtstruktur und Zeolithe in Betracht kommen. Besonders geeignet sind Fällungskatalysatoren, die durch Mischen wäßriger Lösungen von Nickelsalzen und Silicaten, z.B. von Natriumsilicat und Nickelnitrat, und gegebenenfalls weiteren Bestandteilen, wie Aluminiumsalzen, z.B. Aluminiumnitrat, und Calcinieren erhältlich sind.

Besonders bevorzugt sind Katalysatoren, die im Wesentlichen aus NiO, SiO₂, TiO₂ und/oder ZrO₂ sowie gegebenenfalls Al₂O₃ bestehen. Am meisten bevorzugt ist ein Katalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und bis zum Rest zu 100 Gew.-% Siliciumdioxid enthält. Ein solcher Katalysator ist durch Fällung der Katalysatormasse bei pH 5 bis 9 durch Zugabe einer Nickelnitrat enthaltenden wäßrigen Lösung zu einer Alkaliwasserglaslösung, die Titandioxid und/oder Zirkondioxid enthält, Filtrieren, Trocknen und Tempern bei 350 bis 650°C erhältlich. Zur Herstellung dieser Katalysatoren wird im Einzelnen auf die DE-A 43 39 713 verwiesen. Auf die Offenbarung dieser Druckschrift wird vollinhaltlich Bezug genommen.

Das Inkontaktbringen des Butene enthaltenden Kohlenwasserstoffgemisches mit dem Katalysator erfolgt vorzugsweise bei Temperaturen von 30 bis 280, insbesondere 30 bis 140 und besonders bevorzugt von 40 bis 130°C. Es erfolgt vorzugsweise bei einem Druck von 10 bis 300, insbesondere von 15 bis 100 und besonders bevorzugt von 20 bis 80 bar. Der Druck wird dabei zweckmäßigerweise so eingestellt, dass bei der gewählten Temperatur das olefinreiche Kohlenwasserstoffgemisch flüssig oder im überkritischen Zustand vorliegt.

Geeignete Reaktionsapparaturen für das Inkontaktbringen des Butene enthaltenden Kohlenwasserstoffgemisches mit dem heterogenen Katalysator sind z.B. Rohrbündelreaktoren oder Schachtöfen. Aufgrund der geringeren Investitionskosten sind Schachtöfen bevorzugt. Die Dimerisierung kann in einem einzelnen Reaktor durchgeführt werden, wobei der Oligomerisierungskatalysator in einem einzigen oder mehreren Festbetten im Reaktor angeordnet sein kann. Alternativ kann eine Reaktorkaskade aus mehreren, vorzugsweise zwei, hintereinandergeschalteten Reaktoren eingesetzt werden, wobei beim Passieren des bzw. der dem letzten Reaktor der Kaskade vorgeschalteten Reaktors bzw. Reaktoren die Butendimerisierung im Reaktionsgemisch nur bis zu einem Teilumsatz betrieben wird und der gewünschte Endumsatz erst beim Passieren des Reaktionsgemisches durch den letzten Reaktor der Kaskade erzielt wird. Die Butendimerisierung erfolgt vorzugsweise in einem adiabatischen Reaktor oder einer adiabatischen Reaktorkaskade.

Nach dem Verlassen des Reaktors bzw. des letzten Reaktors einer Kaskade werden aus dem Reaktoraustrag die gebildeten Dodecene von den Octenen und gegebenenfalls den höheren Oligomeren und den nicht umgesetzten Butenen und Butanen abgetrennt. Hauptprodukt sind in der Regel die Octene.

Die erhaltenen Dodecene werden im zweiten Verfahrensschritt in an sich bekannter Weise durch Hydroformylierung mit Synthesegas in die um ein C-Atom verlängerten Aldehyde überführt. Die Hydroformylierung von Olefinen zur Herstellung von Aldehyden ist an sich bekannt und z.B. in J. Falbe (Hrsg.): "New Synthesis with Carbon monoxide", Springer Verlag, Berlin, 1980, beschrieben. Die Hydroformylierung erfolgt in Anwesenheit von homogen im Reaktionsmedium gelösten Katalysatoren. Als Katalysatoren werden dabei im Allgemeinen Verbindungen oder Komplexe von Metallen der VIII. Nebengruppe, speziell Co-, Rh-, Ir-, Pd-, Pt- oder Ru-Verbindungen bzw. -Komplexe eingesetzt, die unmodifiziert oder z.B. mit Amin-oder mit phosphinhaltigen Verbindungen modifiziert sein können.

Im Rahmen der vorliegenden Erfindung erfolgt die Hydroformylierung vorzugsweise in Gegenwart eines Cobalt-Katalysators. Sie erfolgt vorzugsweise bei Temperaturen von 120 bis 240, insbesondere 160 bis 200°C und unter einem Synthesegasdruck von 150 bis 400, insbesondere 250 bis 350 bar. Die Hydroformylierung erfolgt vorzugsweise in Gegenwart von Wasser. Das Mischungsverhältnis von Wasserstoff zu Kohlenmonoxid im eingesetzten Synthesegas liegt vorzugsweise im Bereich von 70 : 30 bis 50 : 50 und insbesondere 65 : 35 bis 55 : 45 Vol-%.

Das Cobaltkatalysierte Hydroformylierungsverfahren kann als mehrstufiger Prozess durchgeführt werden, der die folgenden 4 Verfahrensstufen enthält: die Herstellung des Katalysators (Vorcarbonylierung), die Katalysatorextraktion, die Olefinhydroformylierung und die Entfernung des Katalysators aus dem Reaktionsprodukt (Entcobaltung). In der ersten verfahrensstufe, der Vorcarbonylierung, wird ausgehend von einer wäßrigen Cobaltsalzlösung, z.B. Cobaltformiat oder Cobaltacetat, durch Umsetzung mit Kohlenmonoxid und Wasserstoff der für die Hydroformylierung benötigte Katalysatorkomplex (HCo(CO)₄) hergestellt. In der zweiten Verfahrensstufe, der Katalysatorextraktion, wird der in der ersten Verfahrensstufe hergestellte Cobaltkatalysator aus der wäßrigen Phase mit einer organischen Phase, vorzugsweise mit dem zu hydroformylierenden Olefin, extrahiert. Bisweilen ist es zweckmäßig, neben dem Olefin die Reaktions- und Nebenprodukte der Hydroformylierung, sofern sie wasserunlöslich und unter den gewählten Reaktionsbedingungen flüssig sind, zur Katalysatorextraktion einzusetzen. Nach der Phasentrennung wird die mit dem Cobaltkatalysator beladene organische Phase der dritten Verfahrensstufe, der Hydroformylierung, zugeführt. In der vierten Verfahrensstufe, der Entcobaltung, wird die organische Phase des Reaktoraustritts von den Cobaltcarbonylkomplexen in Gegenwart von komplexfreiem Prozesswasser durch Behandlung mit Sauerstoff oder Luft befreit. Dabei wird der Cobaltkatalysator oxidativ zerstört, und die erhaltenen Cobaltsalze werden in die wäßrige Phase zurückextrahiert. Die erhaltende wäßrige Cobaltsalzlösung aus der Entcobaltung wird in die erste Verfahrensstufe, die Vorcarbonylierung, zurückgeführt. Das erhaltene rohe Hydroformylierungsprodukt kann unmittelbar der Hydrierung zugeführt werden. Alternativ kann daraus in üblicher Weise, z.B. destillativ, eine C₁₃-Aldehydfraktion isoliert werden, die der Hydrierung zugeführt wird.

Die Bildung des Cobaltkatalysators, die Extraktion des Cobaltkatalysators in die organische Phase und die Hydroformylierung der Olefine kann auch in einem einstufigen Prozess im Hydroformylierungsreaktor durchgeführt werden.

Verwendbare Cobaltverbindungen sind beispielsweise Cobalt(II)-chlorid, Cobalt(II)-nitrat, deren Amin- oder Hydratkomplexe, Cobaltcarboxylate, wie Cobaltformiat, Cobaltacetat, Cobaltethylhexanoat, Cobaltnaphthenoat, sowie der Cobaltcaprolactamat-Komplex. Unter den Hydroformylierungsbedingungen bilden sich in situ die katalytisch aktiven Cobaltverbindungen in Form von Cobaltcarbonylen. Es können auch die Carbonylkomplexe des Cobalts, wie Dicobaltoctacarbonyl, Tetracobaltdodecacarbonyl und Hexacobalthexadecacarbonyl eingesetzt werden.

Das bei der Hydroformylierung erhaltene Aldehydgemisch wird zu primären Alkoholen reduziert. Im Allgemeinen erfolgt eine teilweise Reduktion bereits unter den Hydroformylierungsbedingungen, wobei die Hydroformylierung auch so gesteuert werden kann, dass eine im Wesentlichen vollständige Reduktion erfolgt. In der Regel wird jedoch das erhaltene Hydroformylierungsprodukt in einem weiteren Verfahrensschritt mit Wasserstoffgas oder einem Wasserstoff enthaltendem Gasgemisch hydriert. Die Hydrierung findet in der Regel in Gegenwart eines heterogenen Hydrierkatalysators statt. Als Hydrierkatalysator kann ein beliebiger zur Hydrierung von Aldehyden zu primären Alkoholen geeigneter Katalysator verwendet werden. Beispiele geeigneter handelsüblicher Katalysatoren sind Kupferchromit, Cobalt, Cobaltverbindungen, Nickel, Nickelverbindungen, die gegebenenfalls geringe Mengen Chrom oder andere Promotoren enthalten, und Gemische von Kupfer, Nickel und/oder Chrom. Die Nickelverbindungen liegen im Allgemeinen geträgert auf Trägermaterialien wie Tonerde oder Kieselgur vor. Weiterhin können Edelmetall enthaltende Katalysatoren wie Platin oder Palladium verwendet werden.

Die Hydrierung kann in Rieselfahrweise erfolgen, wobei das zu hydrierende Gemisch und das Wasserstoffgas bzw. das wasserstoffhaltige Gasgemisch z.B. im Gleichstrom über ein fest angeordnetes Bett des Hydrierkatalysators geleitet werden.

Die Hydrierung findet vorzugsweise bei einer Temperatur von 50 bis 250, insbesondere 100 bis 150°C und einem Wasserstoffdruck von 50 bis 350, insbesondere 150 bis 300 bar statt. Aus dem bei der Hydrierung erhaltenen Reaktionsaustrag kann die gewünschte Isotridecanolfraktion durch fraktionierte Destillation von den C₈-Kohlenwasserstoffen und höhersiedenden Produkten abgetrennt werden.

Die so erhaltenen, im Sinne der vorliegenden Erfindung besonders bevorzugten Isotridecanole zeichnen sich durch eine charakteristische Isomerenverteilung aus, welche beispielsweise mittel Gaschromatographie näher definiert werden kann. Das Gaschromatogramm lässt sich etwa gemäß Kovacs (Z. Anal. Chem. 181, (1961), Seite 351; Adv. Chromatogr. 1 (1965), Seite 229) mit Hilfe von Retentionsindices unter Verwendung von n-Undecanol, n-Dodecanol und n-Tridecanol als Referenzsubstanzen in drei Retentionsbereiche einteilen:
Bereich 1: Retentionsindex <1180
Bereich 2: Retentionsindex 1180 bis 1217
Bereich 3: Retentionsindex >1217

Im Bereich 1 liegen überwiegend drei- und mehrfach-verzweigte Isotridecanole, im Bereich 2 liegen überwiegend zweifach-verzweigte Isotridecanole und im Bereich 3 liegt überwiegend die Summe aus einfach-verzweigten Isotridecanolen und n-Tridecanol vor. Anhand dieser Methode lässt sich die Zusammensetzung von Isotridecanolen durch Vergleich der Flächen unter den entsprechenden Abschnitten der Gaschromatogramm-Kurven ("Flächen-%") im Sinne der vorliegenden Erfindung hinreichend genau ermitteln.

An drei- und mehrfach verzweigten Isomeren weisen die erfindungsgemäß besonders bevorzugten Isotridecanole gemäß der beschriebenen Methode von Kovacs im Allgemeinen 20 bis 60, vorzugsweise 25 bis 50, besonders bevorzugt 40 bis 48 und ganz besonders bevorzugt 45 bis 47 Flächen-% auf.

An zweifach verzweigten Isomeren weisen die erfindungsgemäß besonders bevorzugten Isotridecanole gemäß der beschriebenen Methode von Kovacs im Allgemeinen 10 bis 50, vorzugsweise 20 bis 45, besonders bevorzugt 30 bis 40 und ganz besonders bevorzugt 35 bis 38 Flächen-% auf.

An einfach verzweigten Isomeren und n-Tridecanol weisen die erfindungsgemäß besonders bevorzugten Isotridecanole gemäß der beschriebenen Methode von Kovacs im Allgemeinen 5 bis 30, vorzugsweise 10 bis 25, besonders bevorzugt 15 bis 20 und ganz besonders bevorzugt 17 bis 19 Flächen-% auf.

Die Dichte der besonders bevorzugten Isotridecanole liegt im Allgemeinen bei 0,8 bis 0,9, vorzugsweise bei 0,82 bis 0,86 und besonders bevorzugt bei 0,84 bis 0,845 g/cm³. Ihr Brechungsindex n_{D}²⁰ liegt im Allgemeinen bei 1,4 bis 1,5, vorzugsweise bei 1,44 bis 1,46 und besonders bevorzugt bei 1,446 bis 1,45. Ihre Viskosität liegt im Allgemeinen bei 30 bis 40, vorzugsweise bei 32 bis 38 und besonders bevorzugt bei 34 bis 35,5 mPas. Ihr Siedebereich liegt im Allgemeinen bei 240 bis 280, vorzugsweise bei 250 bis 275 und besonders bevorzugt bei 260 bis 270°C.

Die Herstellung der erfindungsgemäßen Diester der Phthalsäure erfolgt in an sich bekannter Weise durch Veresterung von Phthalsäure oder einem reaktiven Phthalsäurederivat wie Phthalsäureanhydrid oder Phthalsäuredichlorid, mit einem Gemisch aus mindestens einem Decanol und mindestens einem Tridecanol. Vorzugsweise wird die Phthalsäure oder das reaktive Phthalsäurederivat mit einem molaren Überschuß der Alkohole, insbesondere einem molaren Überschuß von 5 bis 30 %, und vorzugsweise in Gegenwart einer Lewis-Säure als Veresterungskatalysator, etwa eines Dialkyltitanats, z.B. Isopropylbutyltitanat, oder einer Brönsted-Säure wie Methansulfonsäure oder Schwefelsäure, umgesetzt.

Die Umsetzung der Phthalsäure oder des reaktiven Phthalsäurederivats mit dem Gemisch aus mindestens einem Decanol und mindestens einem Tridecanol findet im Allgemeinen bei Reaktionstemperaturen von 150 bis 300 und vorzugsweise 200 bis 250°C statt.

Es wird vorzugsweise bei einem molaren Überschuss an dem Gemisch aus mindestens einem Decanol und mindestens einem Tridecanol bezogen auf Phthalsäure oder das reaktive Phthalsäurederivat von 20 bis 30, vorzugsweise um 25 % gearbeitet.

Zur fortlaufenden Entfernung von bei der Veresterung entstehendem Wasser kann während der Umsetzung ein Inertgas, etwa Stickstoff, durch das Reaktionsgemisch geleitet werden.

Nach beendeter Umsetzung arbeitet man das Reaktiongemisch auf das erfindungsgemäße Gemisch von Estern der Phthalsäure auf. Im Allgemeinen geht man dabei so vor, dass man zunächst vor allem organische Verunreinigungen und gegebenenfalls nicht umgesetztes Decanol und/oder Tridecanol weitestgehend abtrennt. Dies erfolgt vorzugsweise durch Abdestillieren des Alkoholüberschusses im Vakuum, Die Destillation wird im Allgemeinen bei einer Temperatur von 150 bis 220 und vorzugsweise 180 bis 200°C und einem Druck von im Allgemeinen 10 bis 100, vorzugsweise 40 bis 60 mbar durchgeführt.

In der Regel wird das rohe Estergemisch anschließend mit einer wässrigen Lauge, z.B. 1 Gew.-prozentiger Natronlauge versetzt und durchmischt, wodurch der Halbester und der Katalysator (gegebenenfalls hydrolysiert und) neutralisiert werden. Dabei bilden sich normalerweise eine wässrige Phase, die abgetrennt wird, und eine organische Phase. Hieran kann sich eine Wasserwäsche der organischen Phase anschließen.

Zur weiteren Reinigung wird das säurefreie und gewaschene Estergemisch vorzugsweise durch Strippen mit Inertgas oder WasserDampf von organischen Verunreinigungen und Restalkoholen befreit. In einer typischen Ausführungsform eines solchen Strippvorganges führt man das Rohprodukt der Strippkolonne am Kopf oder in der Nähe des Kopfes zu und leitet im Gegenstrom Stickstoff oder Wasser-Dampf durch die Kolonne. Der Strippvorgang wird im Allgemeinen bei einer Vorlauftemperatur des rohen Produktes von 130 bis 220 vorzugsweise 170 bis 190°C und einem Druck von 10 bis 100, vorzugsweise 20 bis 40 mbar durchgeführt. Überschreitet die Strippdauer bei diskontinuierlicher Verfahrensführung in Gegenwart von Wasser im Reaktionsgemisch 2 Stunden, so steigt in der Regel die Säurezahl des Produktes in unerwünschter Weise an. Entstandene Säure muss dann erneut neutralisiert und das Wasser aus der Neutralisation abgetrennt werden.

Das gereinigte Estergemisch kann dann bei erhöhter Temperatur im Vakuum mittels Durchleiten eines N₂-Stroms getrocknet und gegebenenfalls durch Inkontaktbringen mit einem Adsorptionsmittel wie Aktivkohle oder Bleicherde weiter gereinigt werden.

Im erfindungsgemäß eingesetzten Alkoholgemisch liegen die Decanole zu den Tridecanolen im molaren Verhältnis von im Allgemeinen 95 : 5 bis 5 : 95, vorzugsweise von 85 : 15 bis 15 : 85, besonders bevorzugt von 75 : 25 bis 25 : 75 und ganz besonders bevorzugt von 75 : 25 bis 65 : 35 vor.

Nach allgemeinem chemischem Wissen darf zwar insbesondere aufgrund der unterschiedlichen elektronischen und sterischen Beschaffenheit der Alkohole davon ausgegangen werden, dass nicht alle erfindungsgemäß für die Herstellung der Estergemische eingesetzten Alkohole die gleiche Reaktivität gegenüber der Phthalsäure oder dem reaktiven Derivat der Phthalsäure haben. In der Regel spiegelt sich jedoch im Estergemisch für praktische Zwecke hinreichend genau die Zusammensetzung des für die Herstellung des Estergemischs verwendeten Alkoholgemisches wieder.

Die auf diese Weise hergestellten erfindungsgemäßen Estergemische enthalten vorzugsweise Diester der Phthalsäure mit je einer Decanol- und einer Tridecanoleinheit und daneben solche mit zwei Decanoleinheiten und mit zwei Tridecanoleinheiten. Setzt man ein Alkoholgemisch ein, welches zu 60 bis 70 Gew.-% aus Decanolen und zu 30 bis 40 Gew.-% Tridecanolen besteht, so kommt man in der Regel zu Produkten folgender Zusammensetzung: 38 bis 45 Gew.-% Diester mit zwei C₁₀-Alkoholen, 40 bis 48 Gew.-% Diester mit je einem C₁₀- und einen C₁₃-Alkohol und 12 bis 15 Gew.-% Diester mit zwei C₁₃-Alkoholen.

Mit dem erfindungsgemäßen Verfahren und den beschriebenen Reinigungsoperationen lassen sich in der Regel Estergemische mit Estergehalten von mehr als 99,5 Gew.-% herstellen.

Bei Verwendung von Gemischen aus isomeren Decanolen und isomeren Tridecanolen ist eine Vielzahl von Estergemischen zugänglich. Diese Estergemische sind allesamt von der vorliegenden Erfindung umfasst. Sie setzen sich aus Phthalsäure-Estern zusammen, die sich demgemäß sowohl hinsichtlich der Kohlenstoffzahl der Alkoholeinheiten in den Estergruppierungen als auch hinsichtlich der Verzweigungen der Alkylketten innerhalb dieser Alkoholeinheiten unterscheiden können.

Die erfindungsgemäßen Gemische von Diestern besitzen im Allgemeinen eine Dichte zwischen 0,94 und 0,97, vorzugsweise 0,945 und 0,965 und, insbesondere zwischen 0,95 und 0,96 g/cm³, eine Viskosität zwischen 100 und 200, vorzugsweise zwischen 120 und 180 und insbesondere zwischen 130 und 160 mPas, und einen Brechungsindex n_{D}²⁰ zwischen 1,475 und 1,495, vorzugsweise zwischen 1,48 und 1,49 und insbesondere zwischen 1,482 und 1,484.

Die erfindungsgemäßen Gemische von Diestern eignen sich als Weichmacher für Formmassen, insbesondere für Formmassen auf PVC-Basis. Sie sind besonders geeignet zur Herstellung von Weich-PVC-Compounds, die eine geringe Flüchtigkeit der Weichmacher und gleichzeitig sehr gute kälteelastische Eigenschaften aufweisen sollen. Für die Herstellung und Untersuchung der unter Einsatz der erfindungsgemäßen Diester hergestellten Weich-PVC-Compounds wird bevorzugt folgende Methode angewandt:

Ein Gemisch wird hergestellt aus PVC-Pulver, vorzugsweise einem nach dem Suspensionsverfahren hergestelltes PVC-Pulver, und einem erfindungsgemäßen Diester der Phthalsäure als Weichmacher. Gewünschtenfalls können weitere Zusätze wie Stabilisatoren, Gleitmittel, Füllstoffe, Pigmente, Farbstoffe, Flamminhibitoren, Lichtstabilisatoren, Antistatika, Treibmittel und Biostabilisatoren zugefügt werden. Dieses Gemisch wird anschließend auf einem Mischwalzwerk plastifiziert und zu einem sogenannten Walzfell gewalzt. Das Walzfell wird anschließend zu einer Weich-PVC-Folie verpreßt, an der dann die anwendungstechnischen Untersuchungen durchgeführt werden.

Die Charakterisierung der kälteelastischen Eigenschaften von Weich-PVC-Compounds erfolgt vorzugsweise anhand der sogenannten "Kältebruchtemperatur" und der "Torsionssteifheit".

Unter der Kältebruchtemperatur versteht man die Temperatur, bei der ein Weich-PVC-Compound in der Kälte bei mechanischer Belastung erste optisch wahrnehmbare Beschädigungen aufweist. Die Bestimmung der Kältebruchtemperatur erfolgte hierin nach DIN 53372.

Unter der Torsionssteifheit versteht man die Temperatur, bei der eine fest vorgegebene Kraft ein Weich-PVC-Compound um einen bestimmten Winkel verdreht. Die Bestimmung erfolgte hierin nach DIN 53447.

Die Erfindung wird nun anhand nachfolgender Beispiele näher erläutert:

### Beispiele

### Beispiel 1

### Herstellung eines Gemischs isomerer Tridecanole

### Verfahrensschritt 1 (Butendimerisierung)

Die Butendimerisierung wurde in einem adiabatischen Reaktor, bestehend aus zwei Teilreaktoren (Länge: jeweils 4 m, Durchmesser: jeweils 80 cm) mit Zwischenkühlung bei 30 bar kontinuierlich durchgeführt. Als Einsatzprodukt wurde ein Raffinat II mit folgender Zusammensetzung verwendet:

| | |
|---|---|
| i-Butan | 2 Gew.-% |
| n-Butan | 10 Gew.-% |
| i-Buten | 2 Gew.-% |
| 1-Buten | 32 Gew.-% |
| trans-2-Buten | 37 Gew.-% |
| cis-2-Buten | 17 Gew.-%. |

Als Katalysator diente ein Material gemäß DE-A 43 39 713, bestehend aus 50 Gew.-% NiO, 12,5 Gew.-% TiO₂, 33,5 Gew.-% SiO₂ und 4 Gew.-% Al₂O₃ in Form von 5 x 5 mm-Tabletten. Die Umsetzung wurde mit einem Durchsatz von 0,375 kg Raffinat II pro Liter Katalysator und Stunde und unter Rückführung des von den gebildeten Oligomeren befreiten Reaktoraustrags mit einem Rückführverhältnis von Rückführmenge zu Raffinat II von 3, einer Eintrittstemperatur am 1. Teilreaktor von 38°C und einer Eintrittstemperatur am 2. Teilreaktor von 60°C durchgeführt. Der Umsatz, bezogen auf die im Raffinat II enthaltenen Butene, lag bei 83,1 %, die Octen-Selektivität betrug 83,3, die Dodecen-Selektivität 12 %. Durch fraktionierte Destillation des Reaktoraustrags wurde die Dodecenfraktion von unumgesetztem Raffinat II, den Octenen und den Hochsiedern abgetrennt.

### Verfahrensschritt 2 (Hydroformylierung und anschließende Hydrierung)

750 g des in Verfahrensschritt 1 hergestellten Dodecen-Gemischs wurden diskontinuierlich in einem Autoklaven mit 0,13 Gew.-% Dicobaltoctacarbonyl (Co₂(CO)₈) als Katalysator unter Zusatz von 75 g Wasser bei 185°C und unter einem Synthesegasdruck von 280 bar bei einem Mischungsverhältnis von H₂ zu CO von 60 : 40 Vol.-% 5 Stunden umgesetzt. Der Verbrauch an Synthesegas, zu erkennen an einem Druckabfall im Autoklaven, wurde durch Nachpressen ergänzt. Nach dem Entspannen des Autoklaven wurde der Reaktoraustrag mit 10 Gew.-%iger Essigsäure durch Einleiten von Luft oxidativ vom Cobaltkatalysator befreit und die organische Produktphase mit Raney-Nickel bei 125°C und einem Wasserstoffdruck von 280 bar 10 h hydriert. Durch fraktionierte Destillation des Reaktoraustrages wurde die Isotridecanolfraktion von den C₁₂-Paraffinen und den Hochsiedern abgetrennt.

Nach der Methode von Kovacs (s.o.) wurde im so hergestellten Isotridecanol der Anteil der drei- und mehrfach verzweigten Isotridecanole zu 45,8, der zweifach verzweigten Isotridecanole zu 36,8 und der der Summe aus einfach verzweigten Isotridecanolen und dem n-Tridecanol zu 17,4 gaschromatographischen Flächenprozenten ermittelt. Dabei wurde wie folgt vorgegangen:

Eine Probe des Isotridecanols wurde mit 1 ml N-Methyl-N-trimethylsilyltrifluoracetamid pro 100 µl Probe 60 Minuten bei 80°C trimethylsilyliert. Für die gaschromatographische Trennung kam eine Trennsäule vom Typ Hewlett Packard Ultra 1 von 50 m Länge auf Basis von zu 100 % methyliertem Silicongummi mit einem Innendurchmesser von 0,32 mm bei einer Filmdicke von 0,33 µm zum Einsatz. Injektor- und Detektortemperatur waren 250°C, die Ofentemperatur betrug 160°C (isotherm). Der Split war 80 ml/min. Als Trägergas diente Stickstoff. Der Vordruck war auf 2 bar eingestellt. Es wurde 1 µl der Probe in den Gaschromatographen eingespritzt, die aufgetrennten Bestandteile wurden mittels FID detektiert. Das Gaschromatogramm wurde zur Auswertung in die Teilbereiche
- Bereich 1: Retentionsindex <1180
- Bereich 2: Retentionsindex 1180 bis 1217
- Bereich 3: Retentionsindex >1217
unterteilt. Als Bezugssubstanzen dienten dabei
- n-Undecanol: Retentionsindex 1100
- n-Tridecanol: Retentionsindex 1200 und
- n-Tridecanol: Retentionsindex 1300.

Die Flächen der Tridecanolpeaks wurden auf 100 Flächenprozent normiert.

Die Dichte des Isotridecanols betrug 0,843 g/cm³, der Brechungsindex n_{D}²⁰ 1,448, die Viskosität 34,8 mPas und der Siedebereich 261 bis 267°C.

### Beispiel 2

Herstellung und Prüfung eines erfindungsgemäßen Diesters aus Phthalsäure und einem Gemisch von Decanolen und Tridecanolen im molaren Verhältnis 70 : 30

307,81 g des Isotridecanols aus Beispiel 1 und 569,09 g eines Gemischs von 2-Propylheptanol und 4-Methyl-2-propylhexanol im Gewichtsverhältnis 89 : 11 (20 % Alkoholüberschuß bezüglich Phthalsäureanhydrid) wurden mit 316,98 g Phthalsäureanhydrid und 0,41 g Isopropylbutyltitanat als Katalysator in einem 2 1-Autoklaven unter N₂-Einperlung (10 1/h) bei einer Rührgeschwindigkeit von 500 U/min und einer Reaktionstemperatur von 230°C umgesetzt. Das gebildete Reaktionswasser wurde fortlaufend mit dem N₂-Strom aus dem Reaktionsgemisch entfernt. Die Reaktionszeit betrug 180 min. Anschließend wurde der Alkohol-Überschuß bei einem Vakuum von 50 mbar abdestilliert. 1000 g des Roh-Phthalates wurden zur Entfernung von sauren Nebenkomponenten, z.B. nicht vollständig veresterter Phthalsäure, mit 150 ml 0,5 Gew.-%-iger Natronlauge durch 10-minütiges Rühren bei 80°C neutralisiert. Es bildete sich ein Zwei-Phasen-Gemisch mit einer oberen organischen Phase und einer unteren wäßrigen Phase (Ablauge mit hydrolysiertem Katalysator). Die wäßrige Phase wurde abgetrennt und die organische Phase zweimal mit 200 ml Wasser nachgewaschen. Zur weiteren Reinigung wurde das neutralisierte und gewaschene Phthalat mit Wasserdampf bei 180°C und 50 mbar Vakuum 2 h behandelt und dadurch Leichtsieder entfernt. Das gereinigte Phthalat wurde dann 30 min bei 150°C/50 mbar mittels Durchleiten eines N₂-Stroms (2 1/h) getrocknet, anschließend mit Aktivkohle 5 min verrührt und bei einer Temperatur von 80°C über eine Nutsche mit Filterhilfsmittel "Supra-Theorit 5" abgesaugt.

Das so erhaltene Gemisch von Diestern hatte eine Dichte von 0,957 g/cm³, eine Viskosität von 138 mPas, einen Brechungsindex n_{D}²⁰ von 1,483, eine Säurezahl von 0,027 mg KOH/g, einen Wassergehalt von 0,029 Gew.-% und eine Reinheit nach GC von 99,83 %.

150 g Suspensions-PVC vom Typ "Vinoflex S 7114" (Firma Solvin), 100 g des erfindungsgemäßen Phthalates und 3 g Ba/Zn-Stabilisator vom Typ "Lankromark LZB 753" wurden mit einem Handmixer bei Raumtemperatur vermischt. Die Mischung wurde anschließend auf einem dampfbeheizten Labormischwalzwerk (Fa. Collin, Typ "150") plastifiziert und zu einem Walzfell verarbeitet. Die Temperatur der beiden Walzen betrug jeweils 170°C; die Drehzahlen lagen bei 15 Upm (vordere Walze) und 12 Upm (hintere Walze); die Walzzeit betrug 5 Minuten. Man erhielt so ein Walzfell mit einer Dicke von 0,55 mm. Das abgekühlte Walzfell wurde anschließend bei einer Temperatur von 180°C und einem Druck von 220 bar innerhalb 400 s auf einer Presse vom Typ "400 P" der Fa. Collin zu einer weich-PVC-Folie mit einer Dicke von 0,50 mm verpresst.

An dieser weich-PVC-Folie wurde anschließend die Flüchtigkeit des Weichmachers nach folgender Methode bestimmt:

An der Decke des Schrankinneren eines handelsüblichen Labortrokkenschranks vom Typ Heraeus T 5042 E, dessen Innenraum die Abmessungen 42 (Breite) x 35 (Höhe) x 32 cm (Tiefe) ahatte, wurde eine Probenhalterung für vier senkrecht herunterhängende Folienproben angebracht, Die Achse dieser Probenhalterung wurde von einem Elektromotor mit 2 Umdrehungen/min angetrieben. Die Folienproben hatten die Abmessung 100 x 150 x 0,5 mm. Sie wurden vor dem Versuch gewogen und dann über eine Lochung an der kurzen Seite in die Probenhalterung eingehängt. Die Folienoberkanten hatten dabei einen Abstand von 70 mm zur Schrankdecke. Auf dem Boden des Schrankinneren war ein Luftverteilerkasten angebracht, der mit einer Metall-Sinterplatte vom Typ "Sika B 100" abgedeckt war, und mit dem im Schrank eine laminare Luftströmung erzeugt wurde. Der Luftdurchsatz wurde auf 800 l/h eingestellt, was einer Luftwechselrate von 17 bis 18 Luftwechseln pro Stunde entsprach. Die Temperatur von 130°C wurde mit einem Temperaturfühler überwacht, dessen Spitze in der Schrankmitte, 100 mm unterhalb der Decke positioniert war. Ein Abluftrohr führte aus dem Schrankinneren zu einer Kondensationsvorrichrung für den Weichmacher. Die versuchsdauer betrug 24 Stunden. Danach wurden die vier Folienproben ausgewogen und die mittlere prozentuale Gewichtsabnahme errechnet.

Die Kältebruchtemperatur wurde nach DIN 53372 und die Torsionssteifheit nach DIN 53447 bestimmt. Die Ergebnisse sind in Tabelle 1 zusammengestellt.

### Beispiel 3

Herstellung und Prüfung eines erfindungsgemäßen Diesters aus Phthalsäure und einem Gemisch von Decanolen und Tridecanolen im molaren Verhältnis 50 : 50

505,01 g des Isotridecanols aus Beispiel 1 und 398,89 g eines Gemischs von 2-Propylheptanol und 4-Methyl-2-propylhexanol im Gewichtsverhältnis 89 : 11 wurden gemäß Beispiel 2 mit 311,05 g Phthalsäureanhydrid und 0,41 g Isopropylbutyltitanat als Katalysator umgesetzt.

Das so erhaltene Gemisch von Diestern hatte eine Dichte von 0,953 g/cm³, eine Viskosität von 146,0 mPas, einen Brechungsindex n_{D}²⁰ von 1,483, eine Säurezahl von 0,027 mg KOH/g, einen Wassergehalt von 0,028 Gew.-% und eine Reinheit nach GC von 99,77 %.

Die Herstellung und die Prüfung eines Weich-PVC-Compounds unter Einsatz des erfindungsgemäßen Phthalates erfolgte analog zu Beispiel 2. Die Ergebnisse sind in Tabelle 1 zusammengestellt.

### Beispiel 4

Herstellung und Prüfung eines erfindungsgemäßen Diesters aus Phthalsäure und einem Gemisch von Decanolen und Tridecanolen im molaren Verhältnis 30 : 70

740,68 g des Isotridecanols aus Beispiel 1 und 250,73 g eines Gemischs von 2-Propylheptanol und 4-Methyl-2-propylhexanol im Gewichtsverhältnis 89 : 11 wurden gemäß Beispiel 2 mit 325,86 g Phthalsäureanhydrid und 0,41 g Isopropylbutyltitanat als Katalysator umgesetzt.

Das so erhaltene Gemisch von Diestern hatte eine Dichte von 0,950 g/cm³, eine Viskosität von 157,0 mPas, einen Brechungsindex n_{D}²⁰ von 1,483, eine Säurezahl von 0,035 mg KOH/g, einen Wassergehalt von 0,027 Gew.-% und eine Reinheit nach GC von 99,81 %.

Die Herstellung und die Prüfung eines Weich-PVC-Compounds unter Einsatz des erfindungsgemäßen Phthalates efolgte analog zu Beispiel 2. Die Ergebnisse sind in Tabelle 1 zusammengestellt.

### Beispiele 5-7: Vergleichsbeispiele

In Analogie zu den Beispielen 2 bis 4 wurden unter Einsatz vonkommerziell verfügbarem Weichmacher " BASF Palatinol Z" (ein Diisododecylphthalat), "BASF Palatinol 10-P" (ein Di-2-propylheptylphthalat) und "BASF Palatinol CE 5455" (ein Diisotridecylphthalat) Weich-PVC-Folien hergestellt und an diesen Folien die Flüchtigkeit des Weichmachers nach der oben beschriebenen Methode, die Kältebruchtemperatur nach DIN 53372 und die Torsionssteifheit nach DIN 53447 bestimmt. Die Ergebnisse sind in Tabelle 1 zusammengestellt.

**Tabelle 1: Ergebnisse der Anwendungstests mit Weichmachern**

| | Flüchtigkeit [% Gewichtsverlust der Probe] | Kältebruchtemperatur nach DIN 53372 [°C] | Torsionssteifheit nach DIN 53447 [°C] |
|---|---|---|---|
| Beispiel 2, erfindungsgemäß | 0,68 | -41 | -41 |
| Beispiel 3, erfindungsgemäß | 0,54 | -42 | -43 |
| Beispiel 4, erfindungsgemäß | 0,38 | -43 | -44 |
| Beispiel 5, Vergleich: BASF Palatinol Z | 0,9 | -36 | -35 |
| Beispiel 6, Vergleich: BASF Palatinol 10-P | 1,2 | -35 | -38 |
| Beispiel 7, Vergleich: BASF Palatinol CE 5455 | 0,5 | -30 | -37 |

Tabelle 1 zeigt, dass die erfindungsgemäßen Weichmacher hinsichtlich ihrer Flüchtigkeit mit dem besten Vergleichsprodukt - nämlich BASF Palatinol CE 5455, einem Diisotridecylphthalat - vergleichbar sind oder dieses sogar übertreffen. Gegenüber allen drei Vergleichsprodukten (BASF Palatinole der Typen Z, 10-P und CE 5455) zeigen die erfindungsgemäßen Weichmacher außerdem jedoch deutlich verbesserte, d.h. niedrigere, Kältebruchtemperaturen und Torsionssteifheiten.

## Patentansprüche

1. Verfahren zur Herstellung von Gemischen von Diestern der Phthalsäure mit Decanolen und Tridecanolen, **dadurch gekennzeichnet, dass** man Phthalsäure oder ein reaktives Derivat der Phthalsäure mit einem Gemisch aus isomeren Decanolen und isomeren Tridecanolen umsetzt.

2. Verfahren nach Anspruch 1, **dadurch gekennzeichnet, dass** man als Decanol ein Gemisch von isomeren Decanolen verwendet, das überwiegend aus 2-Propylheptanol und 4-Methyl-2-propylhexanol besteht.

3. Verfahren nach Anspruch 1 oder 2, **dadurch gekennzeichnet, dass** man als Tridecanol ein Gemisch isomerer Tridecanole verwendet, die zu 20 bis 60 % drei- und mehrfach-verzweigt, zu 10 bis 50 % zweifach-verzweigt und zu 5 bis 30 % einfachverzweigt und/oder n-Tridecanol sind, wobei die Prozentangaben die gaschromatographisch ermittelten Flächen relativ zur Gesamtfläche über alle im analysierten Gemisch enthaltenen Tridecanole wiedergeben, und wobei die Gemische durch Hydroformylierung und Hydrierung eines Gemisches isomerer Dodecene erhalten werden.

4. Verfahren nach Anspruch 3, **dadurch gekennzeichnet, dass** das bei der Herstellung des Gemisches isomerer Tridecanole verwendete Gemisch isomerer Dodecene erhalten worden ist, indem man ein Butene enthaltendes Kohlenwasserstoffgemisch an einem heterogenen Katalysator, welcher Nickeloxid enthält, umsetzt.

5. Verfahren nach Anspruch 4, **dadurch gekennzeichnet, dass** der heterogene Katalysator, welcher Nickeloxid enthält, als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und bis zum Rest zu 100 Gew.-% Siliciumdioxid enthält.

6. Verfahren nach Anspruch 3, **dadurch gekennzeichnet, dass** man als Tridecanol ein Gemisch isomerer Tridecanole verwendet, bei deren Herstellung gemäß einem der Ansprüche 3 bis 5 die Hydroformylierung in Gegenwart eines Cobalt-Katalysators erfolgt.

7. Verfahren nach den Ansprüchen 1 bis 6, **dadurch gekennzeichnet, dass** das Gemisch aus isomeren Decanolen und isomeren Tridecanolen, das Gemisch isomerer Decanole zu 65 bis 75 mol-% und das Gemisch isomerer Tridecanole zu 35 bis 25 mol-% enthält.

8. Verfahren nach den Ansprüchen 1 bis 4, **dadurch gekennzeichnet, dass** das Gemisch aus isomeren Decanolen und isomeren Tridecanolen in einem molaren Überschuss von 20 bis 30 %, bezogen auf Phthalsäure oder das reaktive Phthalsäurederivat, eingesetzt wird.

9. Gemische von Diestern der Phthalsäure, erhältlich nach dem Verfahren gemäß einem der Ansprüche 1 bis 8.

10. Gemische von Diestern der Phthalsäure mit einem Gemisch isomerer Decanole und einem Gemisch isomerer Tridecanole, enthaltend 38 bis 45 Gew.-% Diester mit zwei C₁₀-Alkoholen, 40 bis 48 Gew.-% Diester mit je einem C₁₀- Alkohol und einen C₁₃-Alkohol und 10 bis 15 Gew.-% Diester mit zwei C₁₃-Alkoholen.

11. Verwendung der Gemische von Diestern der Phthalsäure gemäß den Ansprüchen 9 oder 10 als Weichmacher für Formmassen, insbesondere Formmassen auf PVC-Basis.

## Claims

1. A process for preparing mixtures of diesters of phthalic acid with decanols and tridecanols, which comprises reacting phthalic acid or a reactive derivative of phthalic acid with a mixture made from isomeric decanols and isomeric tridecanols.

2. The process according to claim 1, wherein the decanol used comprises a mixture of isomeric decanols composed mainly of 2-propylheptanol and 4-methyl-2-propylhexanol.

3. The process according to claim 1 or 2, wherein the tridecanol used comprises a mixture of isomeric tridecanols which comprises from 20 to 60% of triply or more-than-triply branched tridecanols, from 10 to 50% of doubly branched tridecanols, and from 5 to 30% of singly branched tridecanols and/or of n-tridecanol, where the percentages give the areas determined by gas chromatography relative to the total area over all of the tridecanols comprised in the mixture analyzed, and where the mixtures are obtained by hydroformylation and hydrogenation of a mixture of isomeric dodecenes.

4. The process according to claim 3, wherein the mixture of isomeric dodecenes used in preparation of the mixture of isomeric tridecanols is obtained by reacting a hydrocarbon mixture comprising butenes on a heterogeneous catalyst which comprises nickel oxide.

5. The process according to claim 4, wherein the heterogeneous catalyst which comprises nickel oxide comprises, as substantial active constituents, from 10 to 70% by weight of nickel oxide, from 5 to 30% by weight of titanium dioxide and/or zirconium dioxide, and from 0 to 20% by weight of aluminum oxide, the remainder, to give 100% by weight, being silicon dioxide.

6. The process according to claim 3, wherein the tridecanol used comprises a mixture of isomeric tridecanols, in the preparation of which according to any of claims 3 to 5 the hydroformylation takes place in the presence of a cobalt catalyst.

7. The process according to any of claims 1 to 6, wherein the mixture made from isomeric decanols and from isomeric tridecanols comprises from 65 to 75 mol% of the mixture made from isomeric decanols and from 35 to 25 mol% of the mixture made from isomeric tridecanols.

8. The process according to any of claims 1 to 4, wherein the mixture made from isomeric decanols and isomeric tridecanols is used in a molar excess of from 20 to 30%, based on phthalic acid or the reactive phthalic acid derivative.

9. A mixture of diesters of phthalic acid, obtainable by the process according to any of claims 1 to 8.

10. A mixture of diesters of phthalic acid with a mixture of isomeric decanols and with a mixture of isomeric tridecanols, comprising from 38 to 45% by weight of diester with two C₁₀ alcohols, from 40 to 48% by weight of diester with in each case one C₁₀ alcohol and one C₁₃ alcohol, and from 10 to 15% by weight of diester with two C₁₃ alcohols.

11. The use of the mixtures of diesters of phthalic acid according to claims 9 and 10 as plasticizers for molding compositions, in particular PVC-based molding compositions.

## Revendications

1. Procédé pour la préparation de mélanges de diesters de l'acide phtalique avec des décanols et des tridécanols, **caractérisé en ce que** l'on fait réagir de l'acide phtalique ou un dérivé réactif de l'acide phtalique avec un mélange de décanols isomères et de tridécanols isomères.

2. Procédé suivant la revendication 1, **caractérisé en ce que** l'on utilise comme décanol un mélange de décanols isomères, qui est constitué essentiellement de 2-propylheptanol et de 4-méthyl-2-propylhexanol.

3. Procédé suivant la revendication 1 ou 2,
**caractérisé en ce que** l'on utilise comme tridécanol un mélange de tridécanols isomères qui sont réticulés trois et plusieurs fois à 20-60 %, réticulés deux fois à 10-50 % et réticulés une seule fois à 5-30 % et/ou sont du n-tridécanol, les indications en pourcentage reflétant les surfaces définies par chromatographie en phase gazeuse relativement à la surface totale sur tous les tridécanols contenus dans le mélange analysé, les mélanges étant obtenus par hydroformylation et hydrogénation d'un mélange de dodécènes isomères.

4. Procédé suivant la revendication 3, **caractérisé en ce que** le mélange de dodécènes isomères utilisé pour la préparation du mélange de tridécanols isomères a été obtenu en faisant réagir un mélange d'hydrocarbures contenant des butènes sur un catalyseur hétérogène qui contient de l'oxyde de nickel.

5. Procédé suivant la revendication 4, **caractérisé en ce que** le catalyseur hétérogène qui contient de l'oxyde de nickel contient, comme composés actifs essentiels, 10 à 70 % en poids d'oxyde de nickel, 5 à 30 % en poids de dioxyde de titane et/ou de dioxyde de zircon, 0 à 20 % en poids d'oxyde d'aluminium et pour le reste jusqu'à 100 % en poids de dioxyde de silicium.

6. Procédé suivant la revendication 3, **caractérisé en ce que** l'on utilise comme tridécanol un mélange de tridécanols isomères pour la préparation desquels, suivant l'une des revendications 3 à 5, l'hydroformylation a lieu en présence d'un catalyseur de cobalt.

7. Procédé suivant les revendications 1 à 6,
**caractérisé en ce que** le mélange de décanols isomères et de tridécanols isomères contient le mélange de décanols isomères à raison de 65 à 75 % molaires et le mélange de tridécanols isomères à raison de 35 à 25 % molaires.

8. Procédé suivant les revendications 1 à 4,
**caractérisé en ce que** le mélange de décanols isomères et de tridécanols isomères est mis en oeuvre dans un excédent molaire de 20 à 30 %, par rapport à l'acide phtalique ou au dérivé réactif de l'acide phtalique.

9. Mélanges de diesters de l'acide phtalique, qui peuvent être obtenus selon le procédé suivant l'une des revendications 1 à 8.

10. Mélanges de diesters de l'acide phtalique avec un mélange de décanols isomères et un mélange de tridécanols isomères contenant 38 à 45 % en poids de diesters avec deux alcools en C₁₀, 40 à 48 % en poids de diesters avec chacun un alcool en C₁₀ et un alcool en C₁₃ et 10 à 15 % en poids de diesters avec deux alcools en C₁₃.

11. Utilisation des mélanges de diesters de l'acide phtalique suivant les revendications 9 ou 10 en tant que plastifiants pour matières à mouler, en particulier des matières à mouler à base de PVC.
